(19) Europäisches Patentamt
European Patent Office
Office européen des brevets

(11) Numéro de publication: **0 005 090**
**B1**

(12) **FASCICULE DE BREVET EUROPEEN**

(21) Numéro de dépôt: **79400190.9**

(22) Date de dépôt: **26.03.79**

(51) Int. Cl.³: **C 07 C 93/04**

(54) **Procédé de préparation de la tris(hydroxy-8-dioxa-3,6 octyl)amine et le composé obtenu par ce procédé**

(30) Priorité: **21.04.78 FR 7811823**

(43) Date de publication de la demande:
**31.10.79 Bulletin 79/22**

(45) Mention de la délivrance du brevet:
**01.10.80 Bulletin 80/20**

(84) Etats contractants désignés:
**BE CH DE FR GB IT NL**

(56) Documents cités:
**EP - A - 0 002 147**
**US - A - 2 293 494**
**JOURNAL OF THE AMERICAN SOCIETY**
**volume 70**
**Septemper 1948, n° 9**
**WASHINGTON D.C. (US)**
**J.B. WRIGHT: "Histamine Antagonists**
**I. Alkamine Ethers, pages 3098—3100**

(73) Titulaire: **RHONE-POULENC INDUSTRIES**
**22, avenue Montaigne**
**F - 75008 Paris (FR)**

(72) Inventeur: **Soula, Gérard**
**33, rue Nungesser**
**F - 69330 Meyzieu (FR)**

(74) Mandataire: **Fabre, Madeleine-France et. al.**
**RHONE POULENC Service brevets Chimie et**
**Polymères B.P. 753**
**F - 75360 Paris Cedex 08/FR**

**EP 0 005 090 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation del la tris(hydroxy-8-dioxa-3,6 octyl)amine et le composé obtenu par ce procédé.

La présente invention a pour objet un procédé de préparation de la tris(hydroxy-8 dioxa-3,6 octyl)amine

Il est connu d'après le brevet anglais n° 364.000 de préparer des éthers hydroxylés d'hydroxylalkylamines de formule:

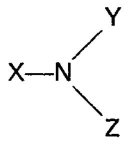

dans laquelle:

— X représente un radical $-(R-O)_n-R_1-OH$ où R et $R_1$ sont des radicaux alcoyle et n un nombre entier;

— Y et Z sont semblables à X ou sont des radicaux alcoyle, cycloalcoyle, etc....

par action d'un oxyde d'alkylène sur un composé aminé contenant au moins un atome d'hydrogène actif en présence d'un diluant aqueux.

Un tel procédé présente l'inconvénient de ne pas être sélectif et de ne conduire qu'à des mélanges d'éthers hydroxylés.

Il est connu d'après le brevet américain n° 2.293.494 de préparer des aminoéthers par action d'un alcoolate de sodium d'alcanolamine (tréthanolamine, polyéthanolamine par exemple) sur une chlorhydrine (chlorhydrine de l'éthylèneglycol, du dièthylèneglycol par exemple). Un tel procédé ne peut être un procédé industriel car il présente l'inconvénient de devoir être réalisé avec d'extrêmes précautions en raison de la forte exothermicité de la réaction mise en jeu; ainsi il est très important de ne pas mettre en présence la totalité de l'alcoolate à faire réagir avec la totalité de la chlorhydrine nécessaire à la réaction; il est conseillé d'ajouter lentement l'alcoolate petit à petit à la chlorhydrine en agitant et en refroidissant constamment mais en maintenant toutefois une température suffisante pour que la réaction ait lieu.

La demanderesse a trouvé un procédé permettant d'obtenir d'une manière sélective la tris(hydroxy-8 dioxa-3,6 octyl)amine de formule:

$$N-[(CH_2-CH_2-O)_2-CH_2-CH_2-OH]_3$$

Le procédé, objet de l'invention, est caractérisé en ce que:

— l'on fait réagir un métal alcalin sur du diéthylèneglycol selon un rapport molaire métal alcalin/diéthylèneglycol, compris entre 1/3 et 1/20

— en ce que l'on condense l'hydroxy-5 oxa-3 pentanolate de métal alcalin formé, en solution dans le diéthylèneglycol, sur une tris (halogénoéthyl)amine choisie parmi le chlorhydrate de tris (chloro-2 éthyl)amine, la tris(chloro-2 éthy)amine ou la tris(bromo-2 éthyl)amine, selon un rapport molaire hydroxy-5 oxa-3 pentanolate de métal alcalin/tris (halogénoéthyl)-amine compris entre 3 et 5 et en ce que l'on récupère la tris(hydroxy-8 dioxa-3,6 octyl)-amine obtenue.

La première étape est réalisée de préférence à partir de sodium ou de potassium avec un rapport molaire métal alcalin/diéthylèneglycol compris entre 1/3 et 1/10. Cette étape est réalisée préférentiellement à température ambiante.

L'étape de condensation peut être réalisée à une température comprise entre 80 et 180, de préférence entre 80 et 150°C pendant 1 à 20 heures, de préférence pendant 1 à 10 heures.

Lorsque l'amine tertiaire halogénée mise en oeuvre est la tris(bromo-2 éthyl)amine ou la tris-(chloro-2 éthyl)amine, le rapport molaire hydroxy-5 oxa-3 pentanolate de métal alcalin/-tris (bromo-2 éthyl)amine ou tris(chloro-2 éthyl)amine, est de préférence compris entre 3 et 4.

Lorsque l'amine tertiaire halogénée mise en oeuvre est le chlorhydrate de tris(chloro-2 éthyl)amine, le rapport molaire hydroxy-5 oxa-3 pentanolate de métal alcalin/chlorhydrate est de préférence compris entre 4 et 5.

Le schéma réactionnel du procédé objet de l'invention, en mettant en oeuvre de sodium à la lère étape et du chlorhydrate de tris(chloro-2 éthyl)amine à l'étape de condensation, est le suivant:

1) $HO-CH_2-CH_2-O-CH_2-CH_2-OH + Na \rightarrow Na^{+-}O-CH_2-CH_2-O-CH_2-CH_2-OH + 1/2\ H_2$

2) $Cl^-H\overset{+}{N}-(CH_2-CH_2-Cl)_3 + 4Na^{+-}O-CH_2-CH_2-O-CH_2-CH_2-OH \rightarrow N-(CH_2-CH_2-O-CH_2-CH_2-O-CH_2-CH_2OH)_3 + 4ClNa$

Le chlorhydrate de tris(chloro-2 éthyl)amine à mettre en oeuvre peut être préparé d'une manière connue à partir de chlorhydrate de triéthanolamine et de chlorure de thionyle selon la méthode décrite par K. WARD dans JACS 57,914,1935 ou par J. P. MASON et D. J. GASCH dans JACS 60,2816,1938.

La tris(chloro-2 éthyl)amine à mettre en oeuvre peut être préparée par décomposition du chlorhydrate de tris(chloro-2 éthyl)amine par du bicarbonate de sodium.

La tris(bromo-2 éthyl)amine à mettre en oeuvre peut être préparée d'une manière connue à partir de la triéthanolamine et du tribromure de phosphore.

La tris(hydroxy-8 dioxa-3,6 octyl)amine preparée selon le procédé de l'invention peut

être utilisée dans les applications classiques des aminopolyols.

L'exemple suivant est donné à titre indicatif et ne peut être considéré comme une limite du domaine et de l'esprit de l'invention.

Exemple 1

Dans un ballon tricol de 2 litres, on introduit 636 g de diéthylèneglycol (6 moles) puis 23 g de sodium (1 mole) par petites portions pendant 3 heures à température ambiante afin de former l'hydroxy-5 oxa-3 pentanolate de sodium.

Après totale disparition du sodium, on ajoute 55 g (0,2 mole) de chlorhydrate de tris(chloro-2 éthyl)amine puis on chauffe le mélange réactionnel à 130°C pendant 4 heures, puis après refroidissement du mélange, on neutralise l'alcoolate en excès par une solution aqueuse d'acide chlorhydrique à 10%. Le diéthylèneglycol en excès est alors distillé et le chlorure de sodium est éliminé par filtration. On obtient ainsi 90 g de tris(hydroxy-8 dioxa-3,6 octyl)amine qui est purifiée par traitement sur charbon actif en solution dans le chlorure de méthylène.

L'analyse élémentaire du produit obtenu est la suivante:

| | — théorie % | mesuré % |
|---|---|---|
| C | 55,38 | 55,40 |
| H | 9,89 | 9,90 |
| N | 3,08 | 3,06 |
| O | 31,65 | 31,64 |

## Revendications

1. Procédé de préparation de la tris(hydroxy-8 dioxa-3,6 octyl)amine, caractérisé en ce que:
— l'on fait réagir un métal alcalin sur du diéthylèneglycol selon un rapport molaire métal alcalin/diéthylèneglycol compris entre 1/3 et 1/20.
— en ce que l'on condense l'hydroxy-5 oxa-3 pentanolate de métal alcalin formé en solution dans du diéthylèneglycol sur une tris(halogénoéthyl)amine choisie parmi le chlorhydrate de tris(chloro-2 éthyl)amine, la tris(chloro-2 éthyl)amine ou la tris(bromo-2 éthyl)amine, selon un rapport molaire hydroxy-5 oxa-3 pentanolate de métal alcalin/tris (halogénoéthyl)amine, compris entre 3 et 5, et en ce que l'on récupère la tris(hydroxy-8 dioxa-3,6 octyl)amine obtenue.

2. Procédé selon la revendication 1 caractérisé en ce que le métal alcalin mis en oeuvre est choisi parmi le sodium et le potassium, et en ce que le rapport molaire métal alcalin/diéthylèneglycol est compris entre 1/3 et 1/10.

3. Procédé selon la revendication 1 caractérisé en ce que l'étape de condensation est réalisée à une température comprise entre 80 et 180°C pendant 1 à 20 heures.

4. Procédé selon la revendication 3 caractérisé en ce que l'étape de condensation est réalisée à une température comprise entre 80 et 150°C pendant 1 à 10 heures.

5. Procédé selon l'une quelconque des revendications 1, 3 ou 4, caractérisé en ce que la tris(halogénoéthyl)amine mise en oeuvre est choisie parmi la tris(chloro-2 éthyl)amine et la tris(bromo-2 éthyl)amine et en ce que le rapport molaire hydroxy-5 oxa-3 pentanolate de métal alcalin/tris(halogénoéthyl)amine est compris entre 3 et 4.

6. Procédé selon l'une quelconque des revendications 1, 3 ou 4, caractérisé en ce que la tris(halogénoéthyl)amine mise in oeuvre est le chlorhydrate de tris(chloro-2 éthyl)amine et en ce que le rapport molaire hydroxy-5 oxa-3 pentanolate de métal alcalin/chlorhydrate est compris entre 4 et 5.

## Patentansprüche

1. Verfahren zur Herstellung von Tris(8-hydroxy-3,6-dioxaoctyl)amin, dadurch gekennzeichnet, daß man ein Alkalimetall auf Diäthylenglykol in einem Molverhältnis von Alkalimetall zu Diäthylenglykol von 1 zu 3 bis 1 zu 20 einwirken läßt und das entstandene, in Diäthylenglykol gelöste Alkali-5-hydroxy-3-oxapentanolat mit einem Tris(halogenäthyl)amin ausgewählt aus der Gruppe Tris(2-chloräthyl)-aminchlorhydrat, Tris(2-chloräthyl)amin oder Tris(2-bromäthyl)amin in einem Molverhältnis von Alkali-5-hydroxy-3-oxapentanolat zu Tris-(halogenäthyl)amin von 3 bis 5 kondensiert und das erhaltene Tris(8-hydroxy-3,6-dioxaoctyl)-amin isoliert.

2. Verfahren nach Anspruch 1, dadurch gekennzeichnet, daß das eingesetzte Alkalimetall ausgewählt wird unter Natrium und kalium und daß das Molverhältnis von Alkalimetall zu Diäthylenglykol 1 zu 3 bis 1 zu 10 beträgt.

3. Verfahren nach Anspruch 1, dadurch gekennzeichnet, daß die Kondensationsstufe bei einer Temperatur zwischen 80 bis 180°C während 1 bis 20 Stunden durchgeführt wird.

4. Verfahren nach Anspruch 3, dadurch gekennzeichnet, daß die Kondensationsstufe bei einer Temperatur zwischen 80 und 150°C während 1 bis 10 Stunden durchgeführt wird.

5. Verfahren nach einem der Ansprüche 1, 2 oder 4, dadurch gekennzeichnet, daß das eingesetzte Tris(halogenäthyl)amin ausgewählt wird unter Tris(2-chloräthyl)amin und Tris(2-bromäthyl)amin und daß das Molverhältnis von Alkali-5-hydroxy-3-oxa-pentanolat zu Tris-(halogenäthyl)amin zwischen 3 und 4 liegt.

6. Verfahren nach einem der Anspruche 1, 3 oder 4, dadurch gekennzeichnet, daß das eingesetzte Tris(halogenäthyl)amin das Tris(2-chloräthyl)amin ist und daß das Molverhältnis von Alkali-5-hydroxy-3-oxapentanolat zu Chlorhydrat zwischen 4 und 5 liegt.

## Claims

1. Process for the preparation of tris-(8-hydroxy-3,6-dioxaoctyl)-amine, characterised in that an alkali metal is reacted with diethylene glycol in a molar ratio alkali metal/diethylene glycol of between 1/3 and 1/20, in that the alkali metal 5-hydroxy-3-oxapentanolate formed is condensed, in solution in diethylene glycol, with a tris-(halogenoethyl)-amine chosen from amongst tris-(2-chloroethyl)-amine hydrochloride, tris-(2-chloroethyl)-amine or tris-(2-bromoethyl)-amine, in a molar ratio alkali metal 5-hydroxy-3-oxapentanolate/tris-(halogenoethyl-amine of between 3 and 5, and in that the resulting tris-(8-hydroxy-3,6-dioxaoctyl)-amine is recovered.

2. Process according to Claim 1, characterised in that the alkali metal employed is chosen from amongst sodium and potassium, and in that the molar ratio alkali metal/diethylene glycol is between 1/3 and 1/10.

3. Process according to Claim 1, characterised in that the condensation step is carried out at a temperature between 80 and 180°C for 1 to 20 hours.

4. Process according to Claim 3, characterised in that the condensation step is carried out at a temperature between 80 and 150°C for 1 to 10 hours.

5. Process according to any one of Claims 1, 3 or 4, characterised in that the tris-(halogenoethyl)-amine employed is chosen from amongst tris-(2-chloroethyl)-amine and tris-(2-bromoethyl)-amine, and in that the molar ratio alkali metal 5-hydroxy-3-oxapentanolate-/tris-(halogenoethyl)-amine is between 3 and 4.

6. Process according to any one of Claims 1, 3 or 4, characterised in that the tris-(halogenoethyl)-amine employed is tris-(2-chloroethyl)-amine hydrochloride, and in that the molar ratio alkali metal 5-hydroxy-3-oxapentanolate/-hydrochloride is between 4 and 5.